# EUROPÄISCHE PATENTANMELDUNG

(11) **EP 4 789 697 A1**
(43) Veröffentlichungstag der Anmeldung: **12.08.2026**
(21) Anmeldenummer: 26157564.1
(22) Anmeldetag: 10.02.2026
(51) Int. Cl.: A61M 1/16

(54) **EXTRAKORPORALE BLUTBEHANDLUNGSVORRICHTUNG MIT VERKÜRZTER DESINFEKTION**

(30) Priorität: 11.02.2025 DE 102025105036
(71) Anmelder: B. Braun Avitum AG, 34212 Melsungen (DE)
(72) Erfinder: JANIK, Waldemar, 34212 Melsungen (DE)
(74) Vertreter: Winter, Brandl - Partnerschaft mbB

(57) **Zusammenfassung**

Die Offenbarung betrifft eine extrakorporale Blutbehandlungsvorrichtung (1) mit: einer Flüssigkeitsbereitstellungseinheit (78, 80), welche eingerichtet ist, eine temperierte oder temperierbare Ausgangsflüssigkeit, vorzugsweise Permeat, bereitzustellen; einer Behandlungsflüssigkeitsbereitstellungseinheit (77), welche eingerichtet ist, eine Behandlungsflüssigkeit, vorzugsweise Dialysierflüssigkeit oder Substituat, bevorzugt unter Verwendung der Ausgangsflüssigkeit, bereitzustellen; einer Schnittstelle (73, 74, 75, 76) an einer Maschinenfront der extrakorporalen Blutbehandlungsvorrichtung (1); einer Bypassleitung (60, 72), welche die Flüssigkeitsbereitstellungseinheit (78, 80) mit der Schnittstelle (73, 74, 75, 76) unter Umgehung der Behandlungsflüssigkeitsbereitstellungseinheit (77) direkt oder indirekt verbindet; und einer Steuereinheit (82), welche eingerichtet ist, eine Desinfektion der extrakorporalen Blutbehandlungsvorrichtung (1) derart auszuführen, dass die temperierte oder temperierbare Ausgangsflüssigkeit von der Flüssigkeitsbereitstellungseinheit (78) über die Bypassleitung (60, 72) und unter Umgehung der Behandlungsflüssigkeitsbereitstellungseinheit (78) der Schnittstelle (73, 74, 75, 76) zugeführt wird.

## Beschreibung

### Technisches Gebiet

Die Offenbarung betrifft eine extrakorporale Blutbehandlungsvorrichtung, ein Verfahren zur Desinfektion einer solchen extrakorporalen Blutbehandlungsvorrichtung und ein System mit einer solchen extrakorporalen Blutbehandlungsvorrichtung.

### Technischer Hintergrund

Vorrichtungen zur extrakorporalen Blutbehandlung sind aus dem Stand der Technik grundsätzlich bekannt. Diese Vorrichtungen dienen der Bereitstellung von Behandlungsflüssigkeit. Die Behandlungsflüssigkeit kann dabei eine Dialysierflüssigkeit und ein Substituat umfassen. Die Behandlungsflüssigkeit kann dabei durch die Aufbereitung von Permeat bzw. Reinstwasser bereitgestellt werden. Dabei wird die Behandlungsflüssigkeit beispielsweise proportioniert, bilanziert und gefiltert, bevor sie an eine Schnittstelle bzw. einen Anschluss zur Weitergabe an eine extrakorporale Blutleitung oder an einen Dialysator geleitet wird. Dabei kann beispielsweise eine Hämodiafiltration oder eine Hämodialyse durchgeführt werden. Nach einer solchen Behandlung werden üblicherweise zumindest die Schnittstellen bzw. Anschlüsse zur Weiterführung, d. h. Zuführung und Rückführung der Behandlungsflüssigkeit gereinigt bzw. desinfiziert. Dieser Desinfektionsvorgang erfolgt mit einer Desinfektionsflüssigkeit. Bei der Desinfektionsflüssigkeit kann es sich im Falle einer Desinfektion mit feuchter Hitze um aufgeheiztes Wasser/ ein aufgeheiztes Permeat handeln. Alternativ oder zusätzlich kann ein Desinfektionsmittel wie Zitronensäure oder Tiutol zugesetzt sein. Dabei werden die Schnittstellen bzw. Anschlüsse mit Desinfektionsflüssigkeit gespült. Dieser Vorgang ist zeitintensiv und verhindert die Verwendung der Vorrichtung für eben diese Zeit.

Aus der US 5895578 A ist ein Verfahren zur Desinfektion einer Dialysemaschine bekannt.

Aus der EP 3765115 B1 ist ein Verfahren zur lokalen Desinfektion einer Dialysemaschinenhydraulik bekannt.

In diesem Zusammenhang hat sich nun herausgestellt, dass ein Bedarf besteht, eine verbesserte extrakorporale Blutbehandlungsvorrichtung bereitzustellen. Insbesondere besteht ein Bedarf, eine extrakorporale Blutbehandlungsvorrichtung mit verkürzter Desinfektion bzw. Desinfektionszeit bereitzustellen.

### Zusammenfassung der vorliegenden Offenbarung

Es ist daher die Aufgabe der vorliegenden Offenbarung, die Nachteile aus dem Stand der Technik zu vermeiden oder wenigstens zu verringern und insbesondere eine extrakorporale Blutbehandlungsvorrichtung, ein Verfahren zur Desinfektion einer solchen extrakorporalen Blutbehandlungsvorrichtung und ein System mit einer solchen extrakorporalen Blutbehandlungsvorrichtung bereitzustellen, mit denen eine zeitlich verkürzte Desinfektion der Schnittstellen bzw. Anschlüsse ermöglicht werden kann.

Die Aufgabe der vorliegenden Offenbarung wird durch eine extrakorporale Blutbehandlungsvorrichtung mit den Merkmalen des Anspruchs 1 sowie durch ein Verfahren zur Desinfektion einer solchen extrakorporalen Blutbehandlungsvorrichtung und ein System mit einer solchen extrakorporalen Blutbehandlungsvorrichtung mit den Merkmalen der nebengeordneten Ansprüche 14 und 16 gelöst. Vorteilhafte Ausgestaltungen sind Gegenstand der Unteransprüche und/oder werden nachfolgend erläutert.

Ein erster Aspekt der vorliegenden Offenbarung betrifft eine extrakorporale Blutbehandlungsvorrichtung mit: einer Flüssigkeitsbereitstellungseinheit, welche eingerichtet ist, eine temperierte oder temperierbare Ausgangsflüssigkeit, vorzugsweise Permeat, bereitzustellen; einer Behandlungsflüssigkeitsbereitstellungseinheit, welche eingerichtet ist, eine Behandlungsflüssigkeit, vorzugsweise Dialysierflüssigkeit oder Substituat, bevorzugt unter Verwendung der Ausgangsflüssigkeit, bereitzustellen, wobei die Behandlungsflüssigkeitsbereitstellungseinheit bevorzugt eine Vielzahl von Komponenten, darunter zumindest einen Filter, insbesondere Ultrafilter, aufweist; einer Schnittstelle bzw. einem Anschluss an einer Maschinenfront der extrakorporalen Blutbehandlungsvorrichtung; einer Bypassleitung, welche die Flüssigkeitsbereitstellungseinheit mit der Schnittstelle bzw. dem Anschluss unter Umgehung der Behandlungsflüssigkeitsbereitstellungseinheit direkt oder indirekt verbindet; und einer Steuereinheit, welche eingerichtet ist, eine Desinfektion der extrakorporalen Blutbehandlungsvorrichtung derart auszuführen, dass die temperierte oder temperierbare Ausgangsflüssigkeit von der Flüssigkeitsbereitstellungseinheit über die Bypassleitung und unter Umgehung der Behandlungsflüssigkeitsbereitstellungseinheit der Schnittstelle bzw. dem Anschluss zugeführt wird, so dass bevorzugt die temperierte oder temperierbare Ausgangsflüssigkeit bei der Desinfektion nicht durch die Komponenten der Behandlungsflüssigkeitsbereitstellungseinheit und somit nicht in und durch den Filter, insbesondere Ultrafilter, fließt.

Der Begriff extrakorporale Blutbehandlung meint vorliegend insbesondere Verfahren, bei denen das Blut außerhalb des Körpers behandelt wird, bevor es zurück in den Körper geleitet wird. Beispiele hierfür sind unter anderem eine Hämodialyse, eine therapeutische Apherese, eine Hämofiltration und eine Hämodiafiltration.

Der Begriff Behandlungsflüssigkeitsbereitstellungseinheit meint vorliegend eine Vorrichtung, die eingerichtet ist, die Behandlungsflüssigkeit, insbesondere Dialysierflüssigkeit und/oder Substituat, bereitzustellen bzw. final aus Permeat bzw. Reinstwasser herzustellen. Die Behandlungsflüssigkeitsbereitstellungseinheit kann dabei eines oder mehr der Folgenden umfassen: einen (Ultra-)Filter, bevorzugt zumindest zwei (Ultra-)Filter, eine Bilanzierungsvorrichtung, eine Pumpe, ein Reservoir für eine Ausgangsflüssigkeit, die zur Behandlungsflüssigkeit weiterverarbeitet wird, eine Schnittstelle für eine solche Ausgangsflüssigkeit, Ventile und Leitungen. Die Behandlungsflüssigkeitsbereitstellungseinheit kann auf mehrere Komponenten verteilt sein. Die Behandlungsflüssigkeitsbereitstellungseinheit bzw. deren Elemente/ Komponenten kann/ können über eine Steuereinheit gesteuert bzw. betrieben werden.

Die Schnittstelle bzw. der Anschluss zur Weiterleitung und Rückführung der Behandlungsflüssigkeit meint vorliegend insbesondere eine Aufnahme, in die ein Schlauch zur Verbindung mit einem Dialysator oder zur Verbindung mit einem extrakorporalen Blutleitungssystem eingesteckt werden kann. Die Schnittstelle bzw. der Anschluss kann weiterhin mit einer Verschlusskappe geschlossen werden, wenn diese/r beispielsweise nicht benötigt wird oder die Schnittstelle/ der Anschluss desinfiziert wird. Die Schnittstelle bzw. der Anschluss ist offenbarungsgemäß an einer Maschinenfront angeordnet. Die Maschinenfront meint vorliegend insbesondere eine Frontfläche der extrakorporalen Blutbehandlungsvorrichtung. Die Frontfläche kann dabei eine beliebige Oberfläche oder mehrere beliebige Oberflächen der extrakorporalen Blutbehandlungsvorrichtung umfassen.

Der Begriff Flüssigkeitsbereitstellungseinheit meint vorliegend insbesondere eine Vorrichtung, die eingerichtet ist, eine temperierte oder temperierbare Ausgangsflüssigkeit, vorzugsweise Permeat/ Reinstwasser, bereitzustellen. Die Flüssigkeitsbereitstellungseinheit kann dabei eines oder mehr der Folgenden umfassen: ein Reservoir für die Ausgangsflüssigkeit, eine Pumpe, ein Heizelement, Ventile, Leitungen etc. Die Flüssigkeitsbereitstellungseinheit bzw. deren Elemente/ Komponenten kann/ können über eine Steuereinheit gesteuert bzw. betrieben werden. Die Ausgangsflüssigkeit kann dabei Permeat und gegebenenfalls Desinfektionsmittel wie Zitronensäure oder Tiutol umfassen. Die Flüssigkeitsbereitstellungseinheit kann auf mehrere Komponenten verteilt sein oder in einer zusammengefasst sein. Es kann grundsätzlich in der offenbarungsgemäßen extrakorporalen Blutbehandlungsvorrichtung eine (einzige) Flüssigkeitsbereitstellungseinheit vorgesehen sein, welche die temperierbare oder temperierte Ausgangsflüssigkeit, insbesondere Permeat/ Reinstwasser sowohl zur Herstellung der Behandlungsflüssigkeit in der Behandlungsflüssigkeitsbereitstellungseinheit bereitstellen kann als auch als Desinfektionsflüssigkeit zur Verwendung im Rahmen einer thermischen Desinfektion, gegebenenfalls im Falle eines Zusatzes eines Desinfektionsmittels im Rahmen einer kombinierten thermischen und chemischen Desinfektion, bereitstellen kann. Alternativ ist es jedoch auch denkbar, dass zwei separate bzw. unabhängige Flüssigkeitsbereitstellungseinheiten vorgesehen sind, von denen eine erste Flüssigkeitsbereitstellungseinheit eine Flüssigkeit zur Herstellung der Behandlungsflüssigkeit bereitstellt und von denen eine zweite Flüssigkeitsbereitstellungseinheit eine (Desinfektions-)Flüssigkeit zur thermischen Desinfektion bzw. gegebenenfalls zur kombinierten thermischen und chemischen Desinfektion bereitstellt.

Der Begriff Bypassleitung meint vorliegend insbesondere eine Leitung, die die Flüssigkeitsbereitstellungseinheit mit der zumindest einen Schnittstelle/ dem zumindest einen Anschluss an der Maschinenfront verbindet. Die Bypassleitung kann dabei mehrere Leitungen umfassen. Die zumindest eine Schnittstelle/ der zumindest eine Anschluss kann mit einer weiteren Leitung, welche mit der Bypassleitung direkt oder indirekt verbunden ist, verbunden sein. Die Bypassleitung ist dabei angeordnet und vorgesehen, dass die Behandlungsflüssigkeitsbereitstellungseinheit umgangen wird, sodass die temperierte oder temperierbare Ausgangsflüssigkeit/ die Desinfektionsflüssigkeit nicht durch die Komponenten der Behandlungsflüssigkeitsbereitstellungseinheit fließen muss und im Rahmen der Desinfektion auch nicht durch die Komponenten der Behandlungsflüssigkeitsbereitstellungseinheit fließt. Die Bypassleitung kann beispielsweise eine separate Leitung umfassen, die auf direkten Weg von der Flüssigkeitsbereitstellungseinheit zu der zumindest einen Schnittstelle/ dem zumindest einen Anschluss führt. Die Bypassleitung kann alternativ Leitungsbereiche umfassen, die die Flüssigkeitsbereitstellungseinheit zur Verbindung mit der zumindest einen Schnittstelle nutzt. Die Leitungsbereiche können Zu- oder Ableitungen zur/ von der Behandlungsflüssigkeitsbereitstellungseinheit darstellen. Die Bypassleitung kann beispielsweise eine Rückleitung/ Rückleitungen umfassen, die die zumindest eine Schnittstelle mit einem Abfluss für die verwendete Behandlungsflüssigkeit verbindet/ verbinden. Mit anderen Worten ausgedrückt ermöglicht die Bypassleitung eine Umgehung der Behandlungsflüssigkeitsbereitstellungseinheit, sodass die großvolumigen Komponenten der Behandlungsflüssigkeitsbereitstellungseinheit, wie (Ultra-)Filter, Bilanziervorrichtung und Proportioniervorrichtung nicht unnötigerweise mit der temperierten (Ausgangs-)Flüssigkeit/ Desinfektionsflüssigkeit während der Desinfektion gefüllt werden müssen. Die Steuereinheit kann insbesondere eingerichtet sein, die ein oder mehrteilige Bypassleitung so zu schalten, dass die zumindest eine Schnittstelle (die Schnittstellen) bzw. der zumindest eine Anschluss (die Anschlüsse) in einer bestimmten Reihenfolge desinfiziert und/oder gleichzeitig desinfiziert werden. In anderen Worten ausgedrückt, kann die Bypassleitung in mehrere Pfade aufgeteilt sein, sodass verschiedene Pfade insbesondere abwechselnd von dem Desinfektionsfluid durchströmt werden können.

Der Begriff Steuereinheit meint vorliegend insbesondere eine Vorrichtung, die eingerichtet ist, Komponenten der Flüssigkeitsbereitstellungseinheit und der Behandlungsflüssigkeitsbereitstellungseinheit wie beispielsweise Pumpen, Ventile, Heizelemente, etc. zu steuern bzw. zu regeln. Die Steuereinheit kann hierzu auf eine Komponente beschränkt oder auf mehrere verteilt sein.

Der Offenbarung liegt die folgende Erkenntnis zugrunde: Extrakorporale Blutbehandlungsvorrichtungen werden üblicherweise zwischen den Einsätzen für die Behandlung unterschiedlicher Patienten an Schnittstellen bzw. Anschlüssen aus hygienischen Gründen desinfiziert. Diese Desinfektion ist zeitaufwändig. Aktuell wird dabei im Wesentlichen die ganze/ gesamte extrakorporale Blutbehandlungsvorrichtung mit temperierter (Desinfektions-)Flüssigkeit gespült. Hierzu werden im Stand der Technik die großvolumigen Komponenten der Behandlungsflüssigkeitsbereitstellungseinheit wie (Ultra-)Filter, Proportioniervorrichtung und Bilanzierungsvorrichtung mit der temperierten Flüssigkeit gefüllt und wieder geleert. Dies ist jedoch nicht zwangsläufig notwendig, da diese Komponenten nicht mit Keimen oder dergleichen in Berührung kommen, da sie immer nur mit frischen Permeat bzw. frischer Dialysierflüssigkeit durchflossen werden. Der unnötige Desinfektionsvorgang bzw. die unnötige Desinfektion dieser Komponenten erhöht die Dauer der Desinfektion. Die Offenbarung schlägt nun vor, bei der Desinfektion die Behandlungsflüssigkeitsbereitstellungseinheit und insbesondere deren großvolumige Komponenten mit einer Bypassleitung zu umgehen, so dass die temperierte (Desinfektions-)Flüssigkeit die zumindest eine Schnittstelle/ den zumindest einen Anschluss schneller erreicht bzw. in anderen Worten kein Füllen der großvolumigen Komponenten der Behandlungsflüssigkeitsbereitstellungseinheit vonnöten ist. Auf diese Weise kann die Zeit für die Desinfektion in vorteilhafterweise Weise reduziert werden und die Verfügbarkeit für die extrakorporale Blutbehandlungsvorrichtung in vorteilhafter Weise erhöht werden. Weiterhin werden vorteilhafterweise weniger Wasser sowie weniger Energie zum Erwärmen des Wassers benötigt. Dadurch können mehr Patienten mit der gleichen Vorrichtung behandelt werden und so die Effizienz der Vorrichtung erhöht werden.

Gemäß einer bevorzugten Ausführungsform kann die extrakorporale Blutbehandlungsvorrichtung weiter eine Entsorgungsschnittstelle zur Entsorgung der verwendeten Ausgangsflüssigkeit umfassen, wobei die Bypassleitung die zumindest eine Schnittstelle mit der Entsorgungsschnittstelle verbindet.

Der Begriff Entsorgungsschnittstelle meint vorliegend insbesondere eine Schnittstelle, die beispielsweise mit einem Entsorgungsbehälter verbunden sein kann. Die Bypassleitung kann dabei eine separate Leitung umfassen, die die verwendete Ausgangsflüssigkeit direkt zur Entsorgungsschnittstelle leitet. Die Bypassleitung kann Ableitungen umfassen, die die Behandlungsflüssigkeitsbereitstellungseinheit zur Entsorgung der verwendeten Behandlungsflüssigkeit verwendet. Letzteres wirkt sich nicht negativ auf die Desinfektionszeit aus, da diese Ableitungen nicht die großvolumigen Komponenten der Behandlungsflüssigkeitsbereitstellungseinheit durchfließen.

Auf diese Weise kann vorteilhafterweise ein vollständiges Desinfektionsleitungssystem bereitgestellt werden.

Gemäß einer bevorzugten Ausführungsform kann die zumindest eine Schnittstelle eines oder mehr der Folgenden umfassen: eine erste Ausgangsschnittstelle zur Weiterleitung der Behandlungsflüssigkeit an einen Dialysator; und/oder eine zweite Ausgangsschnittstelle zur Weiterleitung der Behandlungsflüssigkeit an eine extrakorporale Blutleitung; und/oder eine erste Eingangsschnittstelle zur Rückleitung/ Entsorgung einer verwendeten Behandlungsflüssigkeit nach der Behandlung; und/oder eine zweite Eingangsschnittstelle zur Rückleitung/ Entsorgung einer verwendeten Behandlungsflüssigkeit.

Die erste Ausgangsschnittstelle und die erste Eingangsschnittstelle können beispielsweise zum Transport einer Dialysierflüssigkeit verwendet werden. Die zweite Ausgangsschnittstelle kann beispielsweise für ein Substituat verwendet werden. Die zweite Eingangsschnittstelle kann beispielsweise für die Rückführung bzw. Entsorgung von Behandlungsflüssigkeit, beispielsweise Primingflüssigkeit, verwendet werden. Bei der Verwendung der zweiten Eingangsschnittstelle kann entsprechend das Leitungssystem zum Primen bzw. Entlüften eines extrakorporalen Blutleitungssystems verwendet werden. Dies kann sinnvoll sein, um eine gesundheitsschädliche Infusion von Luft zu verhindern. Somit kann die zweite Eingangsschnittstelle speziell für den Fall des Entlüftens bzw. Primens verwendet werden.

Gemäß einer bevorzugten Ausführungsform kann die Steuereinheit eingerichtet sein, eine Verweildauer der temperierten Flüssigkeit zur Durchführung der Desinfektion zu steuern oder zu regeln.

Der Begriff Verweildauer meint insbesondere die Zeitspanne, in der die Flüssigkeit in bzw. an der Schnittstelle bzw. dem Anschluss vorliegt. Die Steuereinheit kann vorzugsweise eine oder mehrere Pumpen und Ventile für die Leitung der Ausgangsflüssigkeit steuern, sodass diese temperierte Flüssigkeit/ Desinfektionsflüssigkeit an die Schnittstelle/ den Anschluss geleitet wird und sich für eine Verweildauer dort befindet. Dabei kann die Flüssigkeit kontinuierlich die Schnittstelle zur Desinfektion durchfließen oder diskontinuierlich. Die Schnittstelle kann beispielsweise mit der Flüssigkeit einmalig gefüllt werden und dort für eine Verweildauer gehalten werden und dann wieder abgepumpt werden. Die Schnittstelle kann für eine Verweildauer auch kontinuierlich durchströmt werden.

Gemäß einer bevorzugten Ausführungsform kann die Steuereinheit eingerichtet sein, eine Temperatur für die (Ausgangs-)Flüssigkeit zu steuern und/oder zu regeln.

Die Flüssigkeitsbereitstellungseinheit umfasst vorzugsweise ein Heizelement zur Erwärmung der Ausgangsflüssigkeit. Die Steuerungseinrichtung kann über dieses Heizelement die Temperatur der Ausgangsflüssigkeit steuern und/oder regeln. Das Heizelement weist vorzugsweise einen integrierten Temperatursensor hierzu auf. Durch die Bereitstellung einer temperierten, insbesondere erhitzten, Ausgangsflüssigkeit kann die Desinfektion schneller und effizienter durchgeführt werden bzw. die thermische Desinfektion überhaupt erst ermöglicht werden.

Gemäß einer bevorzugten Ausführungsform weist die Flüssigkeitsbereitstellungseinheit ein Heizelement auf, und verbindet die Bypass-Leitung das Heizelement direkt mit der Schnittstelle. Unter einer direkten Verbindung ist dabei zu verstehen, dass sämtliche Komponenten der Behandlungsflüssigkeitsbereitstellungseinheit umgangen werden, und dass die temperierte oder temperierbare Ausgangsflüssigkeit in der Leitung direkt unter geeigneter Schaltung von Zwei-Wege-Ventilen zu der Schnittstelle fließt bzw. strömt. Bevorzugt sind entsprechend in der Leitung zwischen dem Heizelement und der Schnittstelle keine komplexen Mehr-Als-Zwei-Wege Umschaltventile vorgesehen. In anderen Worten ist die Steuereinheit bevorzugt derart eingerichtet, dass bei der Desinfektion die temperierte oder temperierbare Ausgangsflüssigkeit, sobald sie sich in der Leitung zwischen dem Heizelement und der Schnittstelle befindet, auf direktem Weg zur Schnittstelle fließt, ohne dabei auf ihrem Weg zur Schnittstelle in verschiedene bzw. mehrere Leitungsabschnitte abgezweigt zu werden.

Gemäß einer bevorzugten Ausführungsform kann zumindest ein Temperatursensor in der Bypassleitung angeordnet sein, welcher vorzugsweise zur Steuerung oder Regelung der Temperatur in der Bypassleitung verwendet wird.

Durch die Anordnung eines Temperatursensors in der Bypassleitung kann die Temperatur genauer gesteuert bzw. geregelt werden als wenn diese lediglich beim Heizelement erfasst wird. Dies kann vorteilhafterweise die Güte der Desinfektion im Zusammenspiel mit der Verweildauer erhöhen. Weiterhin kann die Desinfektionszeit optimiert werden und gleichzeitig zu hohe Temperaturen, die für die Anlage schädigend sein könnten, vermieden werden. Alternativ oder ergänzend kann ein Temperatursensor an oder nach der ersten Eingangsschnittstelle und/oder der zweiten Eingangsschnittstelle angeordnet sein.

Gemäß einer bevorzugten Ausführungsform kann die Flüssigkeitsbereitstellungseinheit eingerichtet sein, intermittierend temperierte (Ausgangs-)Flüssigkeit bereitzustellen.

Neben der einmaligen und kontinuierlichen Bereitstellung wie oben erwähnt, kann die Schnittstelle bzw. der Anschluss auch intermittierend mit der temperierten (Ausgangs-) Flüssigkeit gefüllt, abgewartet und neu gefüllt werden. Dies kann sich vorteilhaft auf den Desinfektionsvorgang auswirken.

Gemäß einer bevorzugten Ausführungsform kann die Flüssigkeitsbereitstellungseinheit eingerichtet sein, nach Durchführung der Desinfektion, eine Kühlflüssigkeit zur Senkung einer Temperatur der zumindest einen Schnittstelle über die Bypassleitung bereitzustellen.

Die Kühlflüssigkeit kann dabei der Ausgangsflüssigkeit bei einer niedrigeren Temperatur entsprechen. Durch aktive Kühlung mittels Kühlflüssigkeit kann die Dauer für die Desinfektion der Schnittstelle reduziert werden, da die evtl. notwendige Abkühlung schneller abläuft. Auf diese Weise kann die Verfügbarkeit der Anlage erhöht werden. Durch die Verwendung der Bypassleitung kann wiederrum die Durchströmung der großvolumigen Komponenten der Behandlungsflüssigkeitsbereitstellungseinheit umgangen werden. Dies kann sich synergetisch vorteilhaft auf die Effizienz beim Desinfizieren der zumindest einen Schnittstelle auswirken.

Gemäß einer bevorzugten Ausführungsform kann die extrakorporale Blutbehandlungsvorrichtung zumindest eine Versorgungsleitung zur Verbindung der Behandlungsflüssigkeitsbereitstellungseinheit mit der zumindest einen Schnittstelle umfassen.

Durch die Versorgungsleitung kann in vorteilhafter Weise die Grundfunktion der extrakorporalen Blutbehandlungsvorrichtung, nämlich die Bereitstellung der Behandlungsflüssigkeit ermöglicht werden.

Gemäß einer bevorzugten Ausführungsform kann sich die Bypassleitung von der Versorgungsleitung unterscheiden.

Mit anderen Worten ausgedrückt, ist die Bypassleitung bevorzugt eine separate Leitung zur Versorgungsleitung. Dies kann sich vorteilhaft auf die Effizienz auswirken. Beispielsweise kann die Bypassleitung hinsichtlich der Länge optimiert werden, sodass möglichst wenig Volumen zur Desinfektion durchströmt werden muss.

Vorzugsweise kann die Bypassleitung gegenüber einer Umgebung isoliert sein, sodass Wärmeverluste reduziert werden. Dies kann sich vorteilhaft auf die Effizienz der Vorrichtung auswirken.

Gemäß einer bevorzugten Ausführungsform kann die Behandlungsflüssigkeitsbereitstellungseinheit eines oder mehr der Folgenden umfassen: eine Proportioniervorrichtung, einen Zulauf einer Bilanziervorrichtung, zumindest einen (Ultra-)Filter; und/oder kann die Bypassleitung eine Spülleitung zwischen der zweiten Ausgangsschnittstelle und der zweiten Eingangsschnittstelle umfassen; und/oder kann die Bypassleitung eine Spülleitung zwischen der ersten Ausgangsschnittstelle und der ersten Eingangsschnittstelle umfassen.

Der Begriff Proportioniervorrichtung meint vorliegend eine Vorrichtung, die eingerichtet ist, einer Ausgangsflüssigkeit, beispielsweise Permeat, kontrolliert eine basische und eine saure Komponente hinzugegeben, sodass eine Behandlungsflüssigkeit entsteht.

Durch die Spülleitungen kann vorteilhafter ein gemeinsamer Desinfektionskreislauf für mehrere Schnittstellen realisiert werden. Dies kann die Effizienz beim Desinfizieren erhöhen.

Gemäß einer bevorzugten Ausführungsform kann die Flüssigkeitsbereitstellungseinheit eingerichtet sein, nach Durchführung der Desinfektion, eine Kühlflüssigkeit zur Senkung einer Temperatur der zumindest einen Schnittstelle über den zumindest einen Filter und die zumindest eine Versorgungsleitung bereitzustellen.

Dies kann sich vorteilhaft auf die Effizienz beim Desinfizieren der zumindest einen Schnittstelle auswirken.

Gemäß einer bevorzugten Ausführungsform kann die Flüssigkeitsbereitstellungseinheit einen Behälter für Permeat mit Heizelement und zumindest eine Pumpe umfassen.

Ein weiterer Aspekt der vorliegenden Offenbarung betrifft ein Verfahren zur Desinfektion einer extrakorporalen Blutbehandlungsvorrichtung, insbesondere einer oben näher beschriebenen Vorrichtung; umfassend Bereitstellen einer temperierten oder temperierbaren Ausgangsflüssigkeit; und bei der Desinfektion, Zuführen der temperierten oder temperierbaren Ausgangsflüssigkeit von einer Flüssigkeitsbereitstellungseinheit der extrakorporalen Blutbehandlungsvorrichtung über eine Bypassleitung der extrakorporalen Blutbehandlungsvorrichtung und unter Umgehung einer Behandlungsflüssigkeitsbereitstellungseinheit der extrakorporalen Blutbehandlungsvorrichtung an zumindest eine Schnittstelle bzw. einen Anschluss an einer Maschinenfront der extrakorporalen Blutbehandlungsvorrichtung; und vorzugsweise Abführen der Ausgangsflüssigkeit von der zumindest einen Schnittstelle.

Gemäß einer bevorzugten Ausführungsform kann das Verfahren weiter folgende Schritte umfassen: Steuern oder Regeln einer Verweildauer der Ausgangsflüssigkeit; und/oder Steuern oder Regeln einer Temperatur der Ausgangsflüssigkeit; und/oder Sperren einer Versorgungsleitung der extrakorporalen Blutbehandlungsvorrichtung.

Das offenbarungsgemäße Verfahren wird durchgeführt, wenn kein Patient an der extrakorporalen Blutbehandlungsvorrichtung angeschlossen ist. Das offenbarungsgemäße Verfahren ist entsprechend kein Verfahren zur chirurgischen oder therapeutischen Behandlung des menschlichen oder tierischen Körpers und auch kein Diagnostizierverfahren, welches am menschlichen oder tierischen Körper vorgenommen wird.

Ein weiterer Aspekt der vorliegenden Offenbarung betrifft ein System, umfassend eine oben näher beschriebene extrakorporale Blutbehandlungsvorrichtung sowie einen Dialysator und/oder eine extrakorporale Blutleitung bzw. ein extrakorporales Schlauchsystem.

Die Einheiten gemäß einem oder mehreren Ausführungsbeispielen können unter Verwendung von Hardware, Software und/oder einer Kombination davon implementiert werden. Die Einheiten können einteilig oder mehrteilig sein. Hardware-Einheiten können beispielsweise durch Verarbeitungsschaltungen wie einen Prozessor, eine Zentraleinheit (CPU), einen Controller, eine arithmetische Logikeinheit (ALU), einen digitalen Signalprozessor, einen Mikrocomputer, ein feldprogrammierbares Gate-Array (FPGA), ein System-on-Chip (SoC), eine programmierbare Logikeinheit, einen Mikroprozessor oder jede andere Vorrichtung, die in der Lage ist, auf Befehle zu reagieren und diese in einer festgelegten Weise auszuführen, implementiert werden.

Die Einheiten können eine oder mehrere Schnittstellenschaltungen umfassen. In einigen Beispielen können die Schnittstellenschaltungen verdrahtete oder drahtlose Schnittstellen umfassen, die mit einem lokalen Netz (LAN), dem Internet, einem Weitverkehrsnetz (WAN) oder Kombinationen davon verbunden sind. Die Funktionalität einer bestimmten Einheit der vorliegenden Offenbarung kann auf mehrere Einheiten verteilt werden, die über Schnittstellenschaltungen verbunden sind.

Die Einheiten gemäß einem oder mehreren Ausführungsbeispielen können auch ein oder mehrere Speichergeräte umfassen. Bei der einen oder den mehreren Speichervorrichtungen kann es sich um materielle oder nichttransitorische computerlesbare Speichermedien handeln, wie Direktzugriffsspeicher (RAM), Festwertspeicher (ROM), ein permanentes Massenspeichergerät (z. B. ein Festplattenlaufwerk), ein Solid-State-Gerät (z. B. NAND Flash) und/oder jeder andere Datenspeichermechanismus, der Daten speichern und aufzeichnen kann. Die eine Speichervorrichtung oder die mehreren Speichervorrichtungen können zum Speichern von Computerprogrammen, Programmcode, Anweisungen oder eine Kombination davon eingerichtet sein.

Die hier beschriebenen Erläuterungen und Vorteile einzelner Ausführungsformen gelten sinngemäß auch für die anderen Ausführungsformen. Verschiedene beispielhafte Merkmale der Ausführungsformen können offenbarungsgemäß kombiniert werden, wo immer dies technisch sinnvoll und machbar ist.

### Kurzbeschreibung der Figuren

Die vorliegende Offenbarung wird nachfolgend mit Hilfe von Figuren erläutert. Es zeigen.
Figur 1 eine erste schematische Ansicht einer offenbarungsgemäßen extrakorporalen Blutbehandlungsvorrichtung während einer extrakorporalen Blutbehandlung,
Figur 2 eine zweite schematische Ansicht einer offenbarungsgemäßen extrakorporalen Blutbehandlungsvorrichtung während einer Desinfektion,
Figur 3 eine weitere schematische Ansicht einer offenbarungsgemäßen extrakorporalen Blutbehandlungsvorrichtung während einer Desinfektion,
Figur 4 ein Schema für eine zeitliche Abfolge zur Desinfektion einer solchen Vorrichtung, und
Figur 5 eine Darstellung für den Zusammenhang von Zeit und Temperatur bei der Desinfektion.

### Detaillierte Beschreibung von bevorzugten Ausführungsformen

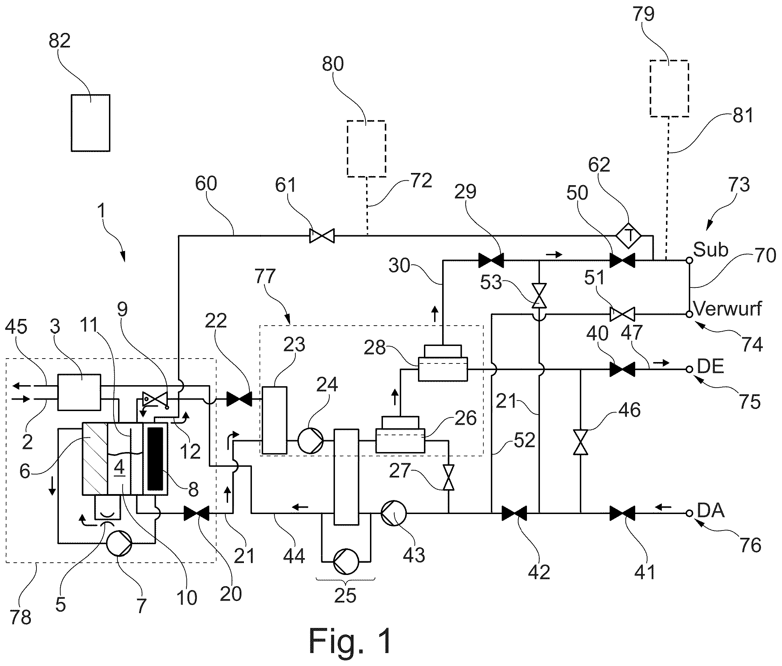

Figur 1 zeigt eine extrakorporale Blutbehandlungsvorrichtung 1.

In den Figuren symbolisieren weiß gefüllte Ventile geschlossene Ventile. Schwarz gefüllte Ventile stehen dagegen für geöffnete Ventile. Die Pfeile neben den Leitungen geben die Flussrichtung an.

Die extrakorporale Blutbehandlungsvorrichtung 1 umfasst eine Behandlungsflüssigkeitsbereitstellungseinheit 77 zur Bereitstellung einer Behandlungsflüssigkeit. Die Behandlungsflüssigkeitsbereitstellungseinheit 77 umfasst vorliegend eine Proportioniervorrichtung 23, einen Zulauf einer Bilanziervorrichtung 25, einen (Ultra-)Filter 26 und einen (Ultra-)Filter 28.

Die extrakorporale Blutbehandlungsvorrichtung 1 umfasst vorliegend eine Flüssigkeitsbereitstellungseinheit 78.

Die Flüssigkeitsbereitstellungseinheit 78 umfasst vorliegend eine Wasserzulauf 2 zur Bereitstellung von Permeat, d.h. hochreines Wasser. Das Permeat wird in einen Vorlaufbehälter 4 geführt. Eine Entgasungspumpe 7 fördert das Permeat aus dem Vorlaufbehälter 4 durch eine Drossel 5 und eine Entgasungskammer 6 zu einer weiteren Kammer samt Heizelement 8, welches das Permeat auf eine gewünschte Solltemperatur aufheizt. Der Unterdruck durch die Drossel 5 führt zum Ausgasen des Permeats. Das erwärmte und entgaste Permeat gelangt durch die Leitung 12 und die Drossel 9 in die Kammer 10, welche mit der Hauptleitung 21 verbunden ist und aus welcher das Permeat zur Herstellung der Dialysierflüssigkeit entnommen werden kann. Steigt der Pegel in der Kammer 10 an, kann das Permeat über die Trennwand 11 zurück in die Kammer 4 fließen. Die Drossel 9 sorgt für einen Aufbau eines Drucks, der benötigt wird, um bei der Verwendung von Natriumhydrogencarbonat (basische Komponente) in Pulverform die zugehörige Kartusche füllen zu können.

Durch das Ventil 22 fließt das erwärmte und entgaste Permeat in die Proportioniervorrichtung 23 der Behandlungsflüssigkeitsbereitstellungseinheit 77. Dort wird dem Permeat, welches hauptsächlich durch die Leitung 21 gelangt, kontrolliert eine basische und eine saure Komponente hinzugegeben, wodurch letztendlich die fertige Dialysierflüssigkeit entsteht. Angetrieben von der Pumpe 24 durchströmt die Dialysierflüssigkeit die Bilanziervorrichtung 25 und wird ein erstes Mal im (Ultra-)Filter 26 gefiltert. Zum Spülen des Filters 26 kann das Ventil 27 geöffnet werden.

Bei geöffnetem Ventil 40 fließt die Dialysierflüssigkeit durch die Leitung 47 über den Dialysierflüssigkeitseingang DE, vorliegend die erste Ausgangsschnittstelle 75, in einen Dialysator (nicht dargestellt) und verlässt den Dialysator anschließend wieder als Dialysat, um über den Dialysatausgang DA, vorliegend die erste Eingangsschnittstelle 76 und über die geöffneten Ventile 41, 42 mittels der Pumpe 43 durch die Leitung 44 zum Wärmetauscher 3 zu fließen und über den Ablauf 45 verworfen zu werden. Die Leitung 47 kann sich zum Teil außerhalb eines Gehäuses der extrakorporalen Blutbehandlungsvorrichtung 1 befinden. Insbesondere kann die Leitung 47 außerhalb des Gehäuses ausgehend von DE in einer (nicht gezeigten) Dialysierflüssigkeitsleitung fortgesetzt werden, die mit dem Dialysator verbunden ist. Die Leitung 44 kann sich zum Teil außerhalb des Gehäuses der extrakorporalen Blutbehandlungsvorrichtung 1 befinden. Insbesondere kann eine an dem Dialysator und an DA angeschlossene Dialysatleitung außerhalb des Gehäuses Teil der Leitung 44 sein. Es wird darauf hingewiesen, dass Leitungen außerhalb des Gehäuses wie etwa eine derartige Dialysierflüssigkeitsleitung oder eine derartige Dialysatleitung zur Vermeidung von Wärmeverlusten bevorzugt isoliert werden. Weiterhin wird darauf hingewiesen, dass im Fall von derartigen außerhalb des Gehäuses vorgesehenen Leitungen eine Spülbrücke bzw. eine Spülleitung mit entsprechenden Anschlüssen an dem Gehäuse vorgesehen sein kann. In diesem Fall können vor dem Spülen bzw. Desinfizieren die mit dem Dialysator verbundenen Enden der Dialysierflüssigkeitsleitung und der Dialysatleitung mit den Anschlüssen der Spülbrücke bzw. Spülleitung verbunden werden, so dass eine direkte Spül- bzw. Desinfizierverbindung zwischen DE und DA über die Dialysierflüssigkeitsleitung, die Spülbrücke bzw. Spülleitung und die Dialysatleitung entstehen kann.

Für die Durchführung einer Hämodiafiltration wird ein Teil der Dialysierflüssigkeit entnommen. Hierzu wird diese Flüssigkeit durch einen zweiten (Ultra-)Filter 28 mittels einer Substitutionspumpe (nicht dargestellt) gefördert. Durch die Leitung 30 und die geöffneten Ventile 29, 50 gelangt das Substituat zum Substitutionsanschluss Sub, vorliegend die zweite Ausgangsschnittstelle 73, und kann ab dort über eine Dilutionsleitung außerhalb des Gehäuses der Vorrichtung 1 einer extrakorporalen Blutleitung zugeführt werden und per Prä- oder Postdilution einem Patienten verabreicht werden.

Beim Füllen bzw. Spülen und/oder Entleeren des extrakorporalen Schlauchsystems kann dieses mit dem Anschluss zum Verwurf, vorliegend die zweite Eingangsschnittstelle 74, verbunden sein. Dabei ist das Ventil 51 geöffnet, so dass ein Fluid über die Leitung 52 abgeführt werden kann. Während einer Hämodiafiltration ist das Ventil 51 geschlossen.

Die Anschlüsse Sub und Verwurf sind insbesondere für Reinigungs- bzw. Desinfektionszwecke über einen Spülkanal 70 miteinander verbunden.

Die extrakorporale Blutbehandlungsvorrichtung 1 kann optional eine separate Flüssigkeitsbereitstellungseinheit 80 aufweisen. Diese Bereitstellungseinheit 80 kann eine Pumpe und einen Behälter mit Heizelement umfassen. Es kann grundsätzlich vorgesehen sein, dass die Flüssigkeitsbereitstellungseinheit 78 lediglich Flüssigkeit zur Herstellung der Behandlungsflüssigkeit bereitstellt, und dass die separate Flüssigkeitsbereitstellungseinheit 80 vorgesehen ist, welche Flüssigkeit für die Desinfektion/ Desinfektionsflüssigkeit bereitstellt. Bevorzugt ist es jedoch, wenn keine separate Flüssigkeitsbereitstellungseinheit 80 vorgesehen ist. In anderen Worten ist es bevorzugt, wenn die Flüssigkeitsbereitstellungseinheit 78 sowohl Flüssigkeit zur Herstellung der Behandlungsflüssigkeit als auch für die Desinfektion bereitstellt.

Die extrakorporale Blutbehandlungsvorrichtung 1 umfasst weiterhin eine Bypassleitung 72, 60, die die zweite Ausgangsschnittstelle 73 bevorzugt mit der Flüssigkeitsbereitstellungseinheit 78, alternativ mit der Flüssigkeitsbereitstellungseinheit 80, zum Durchführen einer Desinfektion verbindet. Die Bypassleitung 72, 60 umgeht dabei die Behandlungsflüssigkeitsbereitstellungeinheit 77, sodass diese bei der Desinfektion nicht mit der Ausgangsflüssigkeit gespült bzw. desinfiziert wird, wenn die zweite Ausgangsschnittstelle 73 desinfiziert wird. Die extrakorporale Blutbehandlungsvorrichtung 1 kann weiterhin optional eine Entsorgungsschnittstelle 79 umfassen, die über eine Bypassleitung 81 mit der zweiten Ausgangsschnittstelle 73 verbunden ist. Die extrakorporale Blutbehandlungsvorrichtung 1 umfasst weiterhin einen Steuereinheit 82, die eingerichtet ist, die Bereitstellung der Behandlungsflüssigkeit und die Bereitstellung der Ausgangsflüssigkeit zu steuern. Die Steuereinheit 82 steuert vorliegend die Verweildauer der Ausgangsflüssigkeit zur Durchführung der Desinfektion sowie die Temperatur der Ausgangsflüssigkeit. Hierzu ist in der Bypassleitung 60 ein Temperatursensor 62 angeordnet. Die Flüssigkeitsbereitstellungseinheit 78 ist vorliegend eingerichtet, die Desinfektionsflüssigkeit einmalig, kontinuierlich oder intermittierend bereitzustellen. Die Flüssigkeitsbereitstellungseinheit 78 ist vorliegend weiter eingerichtet, nach Durchführung der Desinfektion, eine Kühlflüssigkeit zur Senkung einer Temperatur der zweiten Ausgangsschnittstelle 73 über die Bypassleitung 72,60 bereitzustellen. Für den Fall, dass die Bypassleitungen 72 und 81 vorhanden sind, umfassen diese bevorzugt jeweils Ventile (nicht gezeigt).

In Fig. 1 sind die Ventile so geschaltet, dass die Vorrichtung lediglich die Behandlungsflüssigkeiten in Form der Dialysierflüssigkeit und des Substituats bereitstellt. Die Ventilschaltung entspricht somit der Schaltung während der extrakorporalen Blutbehandlung.

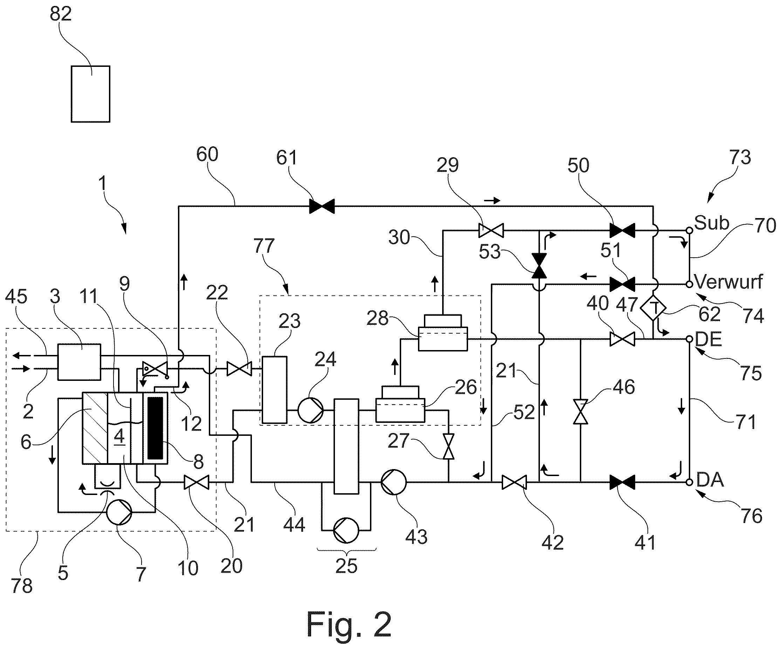

Figur 2 zeigt eine weitere schematische Ansicht der offenbarungsgemäßen extrakorporalen Blutbehandlungsvorrichtung 1. Die extrakorporale Blutbehandlungsvorrichtung 1 befindet sich aktuell im Desinfektionsmodus. Das Heizelement 8 heizt das Permeat auf. Die Ventile 20, 22 sind geschlossen und die Pumpe 24 ist inaktiv. Aufgrund des Überdrucks in Leitung 12 und des geöffneten Ventils 61 kann das erhitzte Wasser durch die Leitung 60 direkt zum Dialysierflüssigkeitsanschluss DE, hier erste Ausgangsschnittstelle 75, gelangen. Leitung 60 ist vorzugsweise isoliert, um Wärmeverluste zu vermeiden. Mit dem Sensor 62 kann die Temperatur erfasst und ggf. vom Heizelement 8 angepasst werden. Über die Spülleitung 71 und die Leitung 21 strömt das erhitzte Fluid zum Substituatanschluss, hier zweite Ausgangsschnittstelle 73 und über den Spülkanal 70 weiter zum Verwurf, hier zweite Eingangsschnittstelle 74, bevor es anschließend in den Ablauf 45 weitergeleitet wird. Auf diese Weise werden die Dialysator-Anschlüsse DE 75 und DA 76 sowie die Anschlüsse für das extrakorporale Blutschlauchsystem Sub 73 und Verwurf 74 von Desinfektionsflüssigkeit (hier erhitztes Permeat) um- bzw. durchgespült. Da das restliche Hydrauliksystem, welches z.B. aufgrund der Proportioniervorrichtung 23 und der Bilanziervorrichtung 25 sowie der beiden Filter 26, 28 über ein hohes Volumen verfügt, überbrückt wird, kann eine verkürzte Heißdesinfektion realisiert werden. Der Temperatursensor 62 kann sich alternativ in der Leitung 52 befinden bzw. kann dort ein zusätzlicher Temperatursensor vorgesehen sein, sodass die Temperatur im gesamten kritischen Bereich gemessen bzw. abgeschätzt werden kann. Der Zustrom der Desinfektionsflüssigkeit durch Leitung 60 kann intermittierend erfolgen. Sinkt die Temperatur im kritischen Bereich, kann das Ventil 61 geöffnet werden, sodass heißere Desinfektionsflüssigkeit nachfließen kann.

Die Desinfektionsflüssigkeit kann durch Überdruck in Leitung 12 zum kritischen Bereich gelangen. Alternativ kann die Pumpe 43 die Desinfektionsflüssigkeit ansaugen. Alternativ kann die Pumpe der Bilanzierungseinheit 25 die Desinfektionsflüssigkeit ansaugen. Alternativ kann die Leitung 60 sich bspw. nach dem Temperatursensor 62 aufteilen. Der erste Teil kann dann in die Substituatleitung 30 münden und der zweite Teil in die Dialysierflüssigkeitsleitung 47. Durch geschickte Ventilschaltung können die zweite Ausgangsschnittstelle 73 und die zweite Eingangsschnittstelle 74 sowie die erste Ausgangsschnittstelle 75 und die erste Eingangsschnittstelle 76 abwechselnd von dem Desinfektionsfluid durchströmt werden, wodurch Wärmeverluste vermieden werden können.

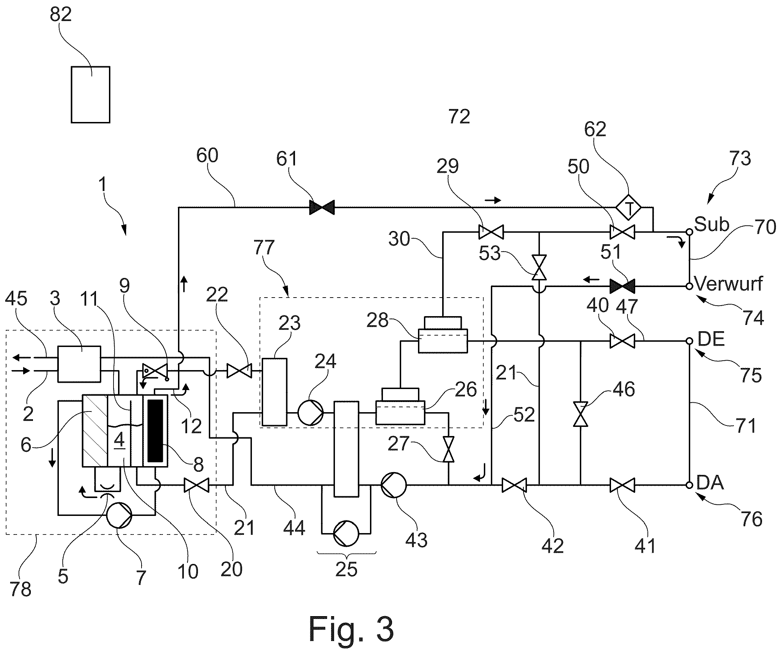

Figur 3 zeigt eine weitere Ansicht einer offenbarungsgemäßen Vorrichtung 1 zur extrakorporalen Blutbehandlung. Hier mündet die Bypassleitung 60 direkt in die Substituatleitung 30. Bei geschlossenem Ventil 50 durchströmt die Desinfektionsflüssigkeit die zweite Ausgangsschnittstelle 73 und gelangt durch die Spülleitung 70 zur zweiten Eingangsschnittstelle 74. In dieser Anordnung werden die erste Ausgangsschnittstelle 75 und die erste Eingangsschnittstelle 76 nicht desinfiziert, da diese evtl. nicht desinfiziert werden müssen, wenn die Behandlungsflüssigkeit während der Behandlung immer von der ersten Ausgangsschnittstelle 75 (durch den Dialysator) zur ersten Eingangsschnittstelle 76 fließt.

In den Figuren 1 bis 3 sind beispielshaft Kreisläufe einer Hämodiafiltrationshydraulik (HDF) gezeigt. Alternativ könnte die Vorrichtung auch für eine Hämodialyse-Hydraulik (HD) verwendet werden. Im Gegensatz zur HDF würden bei einer klassischen HD-Hydraulik im Wesentlichen folgende Komponenten entfallen: 28, 30 29, 50, 73, 70, 74, 51, 53, 21, 52. Wie in Figur 2 dargestellt, würde die Bypassleitung 60 in diesem Fall direkt in die Leitung 47 münden und das Desinfektionsfluid durch 47, 75, 71, 76 und 45 fließen.

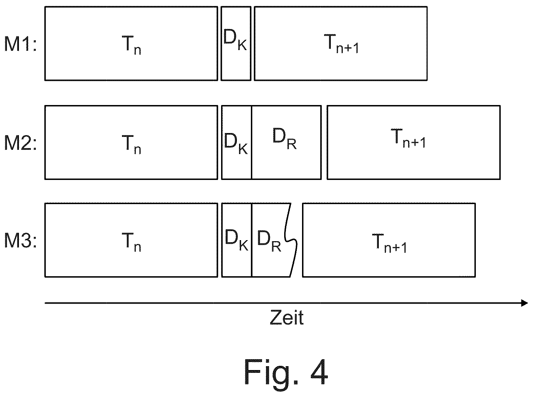

Figur 4 ist ein Schema für eine zeitliche Abfolge zur Desinfektion einer solchen Vorrichtung. In einem Modus M1 nach einer Therapie Tn erfolgt eine kurze Heißdesinfektion des kritischen Bereichs DK, bevor die nächste Therapie Tn + 1 durchgeführt wird. In M2 ist die kurze Heißdesinfektion des kritischen Bereichs DK die erste Sequenz einer Gesamtdesinfektion, in welcher anschließend die Desinfektion der restlichen Hydraulik DR durchgeführt wird. M3 zeigt, wie DR abgebrochen wird, um vorzeitig eine neue Therapie Tn + 1 starten zu können.

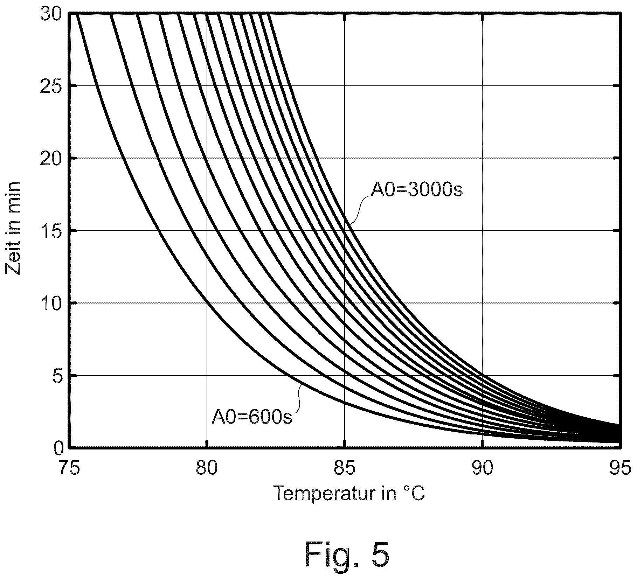

Figur 5 ist eine Darstellung für den Zusammenhang von Zeit und Temperatur bei der Desinfektion. Bei der Desinfektion mit feuchter Hitze können zwei Parameter eingestellt werden. Diese sind die Desinfektionstemperatur und die Desinfektionsdauer. Darüber hinaus spielt die Temperaturempfindlichkeit des betrachteten Mikroorganismus eine Rolle. Der Zusammenhang dieser Parameter ist im A0-Konzept beschrieben. Das A0-Verfahren bewertet den Prozess einer thermischen Desinfektion nach seiner Effektivität. Eine Desinfektion mit einem A0-Wert von 600 s ist für chirurgische Instrumente als Mindestwert angegeben. Dieser Wert sollte auch in der Dialyse bei der Desinfektion des Hydrauliksystems der oben beschriebenen Vorrichtung mindestens erzielt werden können. Vorliegend ist die Einwirkzeit über die Temperatur für verschiedene A0-Werte zwischen 600 s und 3000 s (Abstand von 200 s) dargestellt. Um bspw. einen A0-Wert von 600 s bei einer Temperatur von 90°C zu erzielen, ist eine Einwirkzeit von lediglich 1 min nötig. Ein A0-Wert von 3000 s wird nach 5 min erreicht. Beispielsweise ist die zweite Ausgangsschnittstelle 73 am kritischsten, da hier per Prä- oder Postdilution eine direkte Verbindung zum Blut geschaffen wird. Die zweite Ausgangsschnittstelle 73 kann aus Sicherheitsgründen beispielsweise mit einem höheren Zielwert für A0 desinfiziert werden als beispielsweise die erste Eingangsschnittstelle 76. Bei entsprechender Wahl einer Reihenfolge des Durchströmens, kann vorteilhaft ein Temperaturverlust beispielsweise der ersten Eingangsschnittstelle 76 toleriert werden, da dort ein geringerer A0-Wert als an der zweiten Ausgangsschnittstelle 73 notwendig sein kann.

### Bezugszeichenliste

- 1: Extrakorporale Blutbehandlungsvorrichtung
- 2: Permeatzulauf
- 3: Wärmetauscher
- 4: Vorlaufbehälter
- 5: Drossel
- 6: Entgasungskammer
- 7: Entgasungspumpe
- 8: Heizelement
- 9: Drossel
- 10: Entnahmekammer
- 11: Trennwand
- 12: Leitung
- 20: Ventil Hauptfluss
- 21: dialysierflüssigkeitsführende oder permeatführende Leitung
- 22: dialysatführende Leitung
- 23: Proportioniervorrichtung
- 24: Pumpe
- 25: Bilanziervorrichtung
- 26: Filter
- 27: Ventil
- 28: Filter
- 29: Ventil
- 30: Substituatleitung
- 40: Dialysierflüssigkeitseingangsventil
- 41: Dialysatausgangsventil
- 42: Ventil
- 43: Pumpe
- 44: Dialysatleitung
- 45: Ablauf
- 46: Bypassventil
- 47: Dialysierflüssigkeitsleitung
- 50: Substituatventil
- 51: Verwurfsventil
- 52: Verwurfsleitung
- 53: Bypassventil Substituat
- 60: Leitung Heißfluid
- 61: Ventil
- 62: Temperatursensor
- 70: Spülleitung Substituat
- 71: Spülleitung Dialysat
- 72: Bypassleitung
- 73: zweite Ausgangsschnittstelle
- 74: zweite Eingangsschnittstelle
- 75: erste Ausgangsschnittstelle
- 76: erste Eingangsschnittstelle
- 77: Behandlungsflüssigkeitsbereitstellungseinheit
- 78, 80: Flüssigkeitsbereitstellungseinheit
- 79: Entsorgungsschnittstelle
- 81: Bypassleitung
- 82: Steuereinheit

## Patentansprüche

1. Extrakorporale Blutbehandlungsvorrichtung (1) mit:
einer Flüssigkeitsbereitstellungseinheit (78, 80), welche eingerichtet ist, eine temperierte oder temperierbare Ausgangsflüssigkeit, vorzugsweise Permeat, bereitzustellen;
einer Behandlungsflüssigkeitsbereitstellungseinheit (77), welche eingerichtet ist, eine Behandlungsflüssigkeit, vorzugsweise Dialysierflüssigkeit oder Substituat, bevorzugt unter Verwendung der Ausgangsflüssigkeit, bereitzustellen;
einer Schnittstelle (73, 74, 75, 76) an einer Maschinenfront der extrakorporalen Blutbehandlungsvorrichtung (1);
einer Bypassleitung (60, 72), welche die Flüssigkeitsbereitstellungseinheit (78, 80) mit der Schnittstelle (73, 74, 75, 76) unter Umgehung der Behandlungsflüssigkeitsbereitstellungseinheit (77) direkt oder indirekt verbindet; und
einer Steuereinheit (82), welche eingerichtet ist, eine Desinfektion der extrakorporalen Blutbehandlungsvorrichtung (1) derart auszuführen, dass die temperierte oder temperierbare Ausgangsflüssigkeit von der Flüssigkeitsbereitstellungseinheit (78, 80) über die Bypassleitung (60, 72) und unter Umgehung der Behandlungsflüssigkeitsbereitstellungseinheit (77) der Schnittstelle (73, 74, 75, 76) zugeführt wird.

2. Extrakorporale Blutbehandlungsvorrichtung (1) nach Anspruch 1,
weiter umfassend eine Entsorgungsschnittstelle (45,79) zur Entsorgung der verwendeten Ausgangsflüssigkeit;
wobei die zumindest eine Schnittstelle (73, 74, 75, 76) mit der Entsorgungsschnittstelle (45, 79) verbunden ist.

3. Extrakorporale Blutbehandlungsvorrichtung (1) nach Anspruch 1 oder 2, wobei die zumindest eine Schnittstelle (73, 74, 75, 76) eines oder mehr der Folgenden umfasst:
eine erste Ausgangsschnittstelle (75) zur Weiterleitung der Behandlungsflüssigkeit an einen Dialysator;
eine zweite Ausgangsschnittstelle (73) zur Weiterleitung der Behandlungsflüssigkeit an eine extrakorporale Blutleitung;
eine erste Eingangsschnittstelle (76) zur Rückleitung einer verwendeten Behandlungsflüssigkeit;
eine zweite Eingangsschnittstelle (74) zur Rückleitung einer verwendeten Behandlungsflüssigkeit.

4. Extrakorporale Blutbehandlungsvorrichtung (1) nach einem der vorherigen Ansprüche,
wobei die Steuereinheit (82) eingerichtet ist, eine Verweildauer der Ausgangsflüssigkeit zur Durchführung der Desinfektion zu steuern oder zu regeln.

5. Extrakorporale Blutbehandlungsvorrichtung (1) nach einem der vorherigen Ansprüche,
wobei die Steuereinheit (82) eingerichtet ist, eine Temperatur der Ausgangsflüssigkeit zu steuern oder zu regeln.

6. Extrakorporale Blutbehandlungsvorrichtung (1) nach Anspruch 5,
wobei zumindest ein Temperatursensor (62) zur Steuerung oder Regelung der Temperatur in der Bypassleitung (60, 72) angeordnet ist.

7. Extrakorporale Blutbehandlungsvorrichtung (1) nach einem der vorherigen Ansprüche,
wobei die Flüssigkeitsbereitstellungseinheit (78, 80) eingerichtet ist, intermittierend Ausgangsflüssigkeit bereitzustellen.

8. Extrakorporale Blutbehandlungsvorrichtung (1) nach einem der vorherigen Ansprüche,
wobei die Flüssigkeitsbereitstellungseinheit (78, 80) eingerichtet ist, nach Durchführung der Desinfektion, eine Kühlflüssigkeit zur Senkung einer Temperatur der zumindest einen Schnittstelle (73,74, 75, 76) über die Bypassleitung (60, 72) bereitzustellen.

9. Extrakorporale Blutbehandlungsvorrichtung (1) nach einem der vorherigen Ansprüche, weiter umfassend:
zumindest eine Versorgungsleitung (30, 47) zur Verbindung der Behandlungsflüssigkeitsbereitstellungseinheit (77) mit der zumindest einen Schnittstelle (73,74, 75, 76).

10. Extrakorporale Blutbehandlungsvorrichtung (1) nach Anspruch 9,
wobei die Bypassleitung (60, 72) sich von der Versorgungsleitung (30, 47) unterscheidet.

11. Extrakorporale Blutbehandlungsvorrichtung (1) nach einem der vorherigen Ansprüche,
wobei die Behandlungsflüssigkeitsbereitstellungseinheit (77) eines oder mehr der Folgenden umfasst: eine Proportioniervorrichtung (23), einen Zulauf einer Bilanziervorrichtung (25), zumindest einen Filter (26, 28); und/oder
wobei die Bypassleitung eine Spülleitung (70) zwischen der zweiten Ausgangsschnittstelle (73) und der zweiten Eingangsschnittstelle (74) umfasst; und/oder
wobei die Bypassleitung eine Spülleitung (71) zwischen der ersten Ausgangsschnittstelle (75) und der ersten Eingangsschnittstelle (76) umfasst.

12. Extrakorporale Blutbehandlungsvorrichtung (1) nach Anspruch 9 und 11, wobei die Flüssigkeitsbereitstellungseinheit (78, 80) eingerichtet ist, nach Durchführung der Desinfektion, eine Kühlflüssigkeit zur Senkung einer Temperatur der zumindest einen Schnittstelle (73,74, 75, 76) über den zumindest einen Filter (26, 28) und die zumindest eine Versorgungsleitung (30, 47) bereitzustellen.

13. Extrakorporale Blutbehandlungsvorrichtung (1) nach einem der vorherigen Ansprüche,
wobei die Flüssigkeitsbereitstellungseinheit (78, 80) einen Behälter (8) für Permeat mit Heizelement und zumindest eine Pumpe (7) umfasst.

14. Verfahren zur Desinfektion einer extrakorporalen Blutbehandlungsvorrichtung (1), vorzugsweise einer extrakorporalen Blutbehandlungsvorrichtung nach einem der Ansprüche 1 bis 13; umfassend
Bereitstellen einer temperierten oder temperierbaren Ausgangsflüssigkeit; und
bei der Desinfektion, Zuführen der temperierten oder temperierbaren Ausgangsflüssigkeit von einer Flüssigkeitsbereitstellungseinheit (78, 80) über zumindest eine Bypassleitung (60, 72) und unter Umgehung einer Behandlungsflüssigkeitsbereitstellungseinheit (77) an zumindest eine Schnittstelle (73, 74, 75, 76) an einer Maschinenfront der extrakorporalen Blutbehandlungsvorrichtung (1);
und vorzugsweise, Abführen der Ausgangsflüssigkeit von der zumindest einen Schnittstelle (73, 74, 75, 76).

15. Verfahren nach Anspruch 14, weiter umfassend
Steuern oder Regeln einer Verweildauer der Ausgangsflüssigkeit; und/oder
Steuern oder Regeln einer Temperatur der Ausgangsflüssigkeit; und/oder
Sperren einer Versorgungsleitung (30, 47).

16. System umfassend eine extrakorporale Blutbehandlungsvorrichtung (1) nach einem der Ansprüche 1 bis 13 sowie einen Dialysator und/oder eine extrakorporale Blutleitung.
